# EUROPEAN PATENT SPECIFICATION

(11) **EP 3 941 447 B1**
(45) Date of publication and mention of the grant of the patent: **07.05.2025**
(21) Application number: 20720932.1
(22) Date of filing: 23.03.2020
(51) Int. Cl.: A61K 31/01, A61K 31/015, A61K 31/045, A61K 31/05, A61K 31/11, A61K 31/192, A61K 31/616, A61P 35/00

(54) **EUTECTIC COMPOSITION COMPRISING A NONSTEROIDAL ANTI-INFLAMMATORY DRUG AND A TERPENE FOR USE IN THE TREATMENT OF CANCER**
EUTEKTISCHE ZUSAMMENSETZUNG MIT EINEM NICHTSTEROIDALEN ENTZÜNDUNGSHEMMENDEN ARZNEIMITTEL UND EINEM TERPEN ZUR VERWENDUNG BEI DER BEHANDLUNG VON KREBS
COMPOSITION EUTECTIQUE COMPRENANT UN ANTI-INFLAMMATOIRE NON STÉROÏDIEN ET UN TERPÈNE POUR UTILISATION DANS LE TRAITEMENT DU CANCER

(30) Priority: 21.03.2019 PT 2019115383
(43) Date of publication of application: 26.01.2022
(73) Proprietor: UNIVERSIDADE NOVA DE LISBOA, 1099-085 Lisboa (PT); Association for the Advancement of Tissue Engineering and Cell Based Technologies & Therapies (A4TEC) - Associação, 4710-057 Braga (PT); NOVA.ID.FCT - ASSOCIAÇÃO PARA A INOVAÇÃO E DESENVOLVIMENTO DA FCT, 2829-516 Caparica (PT)
(72) Inventor: MARQUES DA SILVA, Joana Maria, 4805-473 Santa Leocádia Briteiros (PT); VELHINHO PEREIRA, Carolina, 2645-542 Alcabideche (PT); AMARO RODRIGUES, Liliana Andreia, 2640-741 São Miguel De Alcainça (PT); GONÇALVES REIS, Rui Luis, 4250-242 Porto (PT); DE ALMEIDA PAIVA, Alexandre Babo, 1700-011 Lisboa (PT); FIGUEIREDO MATIAS, Ana Alexandra, 2820-036 Charneca Da Caparica (PT); CRUZ DUARTE, Ana Rita, 2820-142 Charneca Da Caparica (PT)
(74) Representative: Patentree
(86) International application number: PCT/IB2020/052708
(87) International publication number: WO 2020/188547

(56) References cited:
- EP-A1- 0 085 544
- WO-A1-2006/096955
- WO-A2-00/25763
- WO-A2-97/48391
- CN-A- 108 096 257
- CN-A- 108 853 123
- CN-B- 102 351 631
- US-A1- 2006 241 175
- US-A1- 2007 224 261
- US-B2- 7 138 394
- YU XIAO ET AL: "D-limonene exhibits antitumor activity by inducing autophagy and apoptosis in lung cancer", ONCOTARGETS AND THERAPY, vol. Volume 11, 1 January 2018 (2018-01-01), pages 1833 - 1847, XP055950806, Retrieved from the Internet <URL:https://www.dovepress.com/getfile.php?fileID=41316> DOI: 10.2147/OTT.S155716
- ARUNASREE ET AL: "Anti-proliferative effects of carvacrol on a human metastatic breast cancer cell line, MDA-MB 231", PHYTOMEDICINE, ELSEVIER, AMSTERDAM, NL, vol. 17, no. 8-9, 1 July 2010 (2010-07-01), pages 581 - 588, XP027053901, ISSN: 0944-7113, [retrieved on 20100518]

## Description

### Technical field

The present disclosure describes a eutectic composition for use in the treatment of cancer, comprising a nonsteroidal anti-inflammatory drug and a terpene as defined in the set of claims.

### Background

Deep eutectic solvents (DES) have emerged in the last decade as a new class of ionic liquid (IL) analogues. Although DES share many characteristics with ILs, the terms are not interchangeable and DES offers several other advantages that turns them into a viable alternative to ILs. Contrary to ILs, DES fully obey the green chemistry metrics being less toxic, often biodegradable and no waste is generated upon their production. Furthermore, DES are cheaper to produce since the raw materials have lower cost and the synthesis is very simple and with high purity as compared with other designer solvents. DES are obtained by mixing two or more components which at certain molar ratio supress the melting point of their chief compounds. This depression in temperature is the result of charge delocalization occurring via hydrogen bonding between the components of the mixture. The properties of DES can be tuned by changing the molar ratio and/or nature of hydrogen bond donor (HBD) and the hydrogen bond acceptor (HBA), which in turn influence the position and the number of the hydrogen bonds. All these attractive features have positioned DES as attractive and advanced designer solvents with a wide range of applications including extraction, carbon dioxide capture, electrochemistry, biocatalysis and biomedical applications. In the biomedical field it has been reported that DES improve the solubility, permeation and absorption of model active pharmaceutical ingredients (API's). When one of the components of the DES is an API the system is called therapeutic deep eutectic solvents (THEDES).

Fatty acids, terpenes and nonsteroidal anti-inflammatory drugs (NSAIDs) have already been reported to have an effect on the inflammation process. Capric acid ("CA"), is known to act against oxidative stress and pro-inflammatory cytokines. Menthol is capable of reducing IL-1β at chronic colonic inflammation. Ibuprofen ("IBU") is a commercialized anti-inflammatory compound also associated with tumour reduction.

The scientific publication by N. González and H. Sumano on "Design of Two Liquid Ibuprofen-Poloxamer-Limonene or Menthol Preparations for Dermal Administration" discloses a method of preparing an ibuprofen-limonene mixture with enhanced transdermal permeation preparation. The enhanced transdermal permeation effect is due to the addition of Poloxamer 407 to the mixture. The scientific publication does not disclose an ibuprofen-limonene mixture whereby the enhanced cell permeation property observed is attributed to the mixture being a deep eutectic system.

Document CN 108186620 A discloses the use of ibuprofen in the preparation of antitumor drugs. Specifically, the document discloses the use of a specific concentration (10 % to 19 %) of ibuprofen as an injection for the treatment of pancreatic cancer. The document does not disclose the use of ibuprofen together with limonene as an anti-tumour drug mixture.

Document US 2006/241175 A1 discloses a liquid eutectic mixture of hydrophobic compounds for the topical delivery of pharmaceutically active ingredients aimed at effective pain control and inflammation treatment.

Document WO 2006/096955 A1 discloses a vehicle for the topical delivery of pharmaceutically active ingredients, such as anti-inflammatory drugs, incorporating a liquid eutectic mixture of hydrophobic components. The eutectic mixture comprises compounds such as camphor, menthol, thymol, resorcinol, phenol, or substituted phenol derivatives, which enhance the solubility of the active ingredients.

Document US 7 138 394 B2 discloses a vehicle for topical delivery containing a liquid eutectic mixture of hydrophobic compounds, enabling effective localized administration of pharmaceutically active ingredients designed for the topical treatment of pain and inflammation.

Document US 2007/224261 A1 discloses a drug formulation comprising an eutectic liquid, which is incorporated into an oral capsule. The eutectic liquid is formed by mixing a pharmaceutically active substance, solid at room temperature, with a biologically tolerated compound.

Document CN 108 096 257 A discloses the use of aspirin in combination with octininib to overcome acquired resistance and increase the sensitivity of non-small cell lung cancer (NSCLC) to oxitinib, providing a new therapeutic approach for treating NSCLC.

Document CN 108 853 123 A describes the use of aspirin in the prevention or treatment of colon cancer.

Document EP 0 085 544 A1 discloses flurbiprofen or ibuprofen, or their C₁₋₈ alkyl esters or pharmacologically acceptable salts for use in the treatment of acute or chronic myelogenous leukemia.

Document WO 97/48391 A2 discloses the use of ibuprofen in the treatment of malignancies like cancer, as well as in the therapeutic and prophylactic treatment of Alzheimer's and Alzheimer's-related diseases.

Document YU XIAO ET AL: "D-limonene exhibits antitumor activity by inducing autophagy and apoptosis in lung cancer", ONCOTARGETS AND THERAPY, vol. Volume 11, 1 January 2018 (2018-01-01), pages 1833-1847, XP055950806 discloses that D-limonene inhibits lung cancer cell growth and tumor progression by inducing apoptosis through autophagy promotion, highlighting its potential as a therapeutic agent for lung cancer.

Document WO 00/25763 A2 discloses a composition of phenol derivatives, particularly thymol, eugenol, hydroxy benzoic acids, their derivatives, and mixtures thereof, for inhibiting the proliferation of tumour cells.

Document CN 102 351 631 B discloses an alpha-pinene extracted from pine needle, its extraction method and application for inhibiting the proliferation of tumor cells.

Document ARUNASREE ET AL: "Anti-proliferative effects of carvacrol on a human metastatic breast cancer cell line, MDA-MB 231", PHYTOMEDICINE, ELSEVIER, AMSTERDAM, NL, vol. 17, no. 8-9, 1 July 2010 (2010-07-01), pages 581-588, XP027053901 describes the anti-tumor effects of carvacrol on human metastatic breast cancer cells (MDA-MB 231), revealing that it induces apoptosis by decreasing mitochondrial membrane potential, releasing cytochrome c, activating caspases, and cleaving PARP.

These facts are disclosed in order to illustrate the technical problem addressed by the present disclosure.

### General Description

This disclosure relates to the thermophysical properties of different therapeutic deep eutectic systems ("THEDES") for use in the treatment or therapy of cancer as defined in the set of claims.

Carcinogenesis is a phenomenon not only restricted to the abnormal growth of cells but also includes angiogenesis and inflammation processes which play an important role in tumour progression. The bioactivity of these emerging THEDES solvents is not yet well explored, nonetheless, literature has already reported cytotoxicity of ammonium- and choline chloride-based DES for several cancer cell lines. This disclosure describes the potential of naturally occurring molecules such as terpenes and fatty acids in anticancer therapies.

Limonene ("LIM") is a cyclic monoterpene studied at preclinical and clinical levels due to its chemopreventive and chemotherapeutic activities against several types of cancer such as lung, breast, gastric and prostate etc. It is generally recognized as safe (GRAS) for use in the food industry as a flavouring agent. Its high lipophilicity contributes to a favourable cellular absorption, specifically at the intestinal level, allowing good bioavailability in the systemic circulation. In colorectal cancer, limonene has been reported to induce apoptosis via mitochondrial pathway and affects the PI3K/Akt signalling pathway (survival and apoptosis).

One of the aspects of the present disclosure is the development of novel THEDES based on LIM due to its well-known anticancer properties. LIM was combined with different compounds, including menthol, IBU and CA due to their well-known anti-inflammatory properties. CA also increases the bioavailability of the API.

An aspect of the present disclosure described a eutectic composition for use in the treatment or therapy of cancer, comprising a nonsteroidal anti-inflammatory drug and a terpene, wherein the composition comprises a combination of ibuprofen and limonene; ibuprofen and menthol, ibuprofen and thymol, or ibuprofen and perillyl alcohol, wherein the molar ratio of anti-inflammatory drug:terpene varies from 1:1 - 1:8.

In an embodiment for better results, the molar ratio of anti-inflammatory drug:terpene may vary from 1:3 - 1:6. Preferably, the molar ratio of anti-inflammatory drug:terpene varies from 1:4 - 1:6.

In an embodiment for better results, the molar ratio of ibuprofen:terpene varies from 1:4 - 1:6.

In an embodiment for better results, the molar ratio of anti-inflammatory drug:limonene varies from 1:4 - 1:6.

In an embodiment for better results, the composition of the present disclosure comprising preferably ibuprofen and limonene, or ibuprofen and perillyl alcohol.

In an embodiment for better results, the molar ratio of ibuprofen:limonene varies from 1:3 - 1:8, preferably from 1:4 - 1:6.

In an embodiment for better results, the ibuprofen concentration is preferably from 0.15-0.75 mM, preferably from 0.25 - 0.5 mM.

In an embodiment for better results, the limonene concentration is preferably from 0.5-3 mM, preferably from 1-2 mM.

In an embodiment, the composition is an injectable composition or an oral composition.

Another aspect of the present disclosure is the use of the eutectic composition of the present subject-matter for use in medicine or as a medicament, or veterinary. Preferably, for use in cancer therapy or treatment.

Another aspect of the present disclosure is the use of the composition as an anti-proliferator for cancer cells.

Another aspect of the present disclosure is a method of inhibiting the proliferation of cancer cells in a subject, the method comprising administering the eutectic composition of the present subject-matter. The references to the methods of treatment by therapy of this disclosure are to be interpreted as references to compounds, pharmaceutical compositions and medicaments of the present disclosure for use in those methods.

In one embodiment, different formulations based on LIM were prepared. The formulations that led to a pasty-like solid at room temperature ("RT", 20°C) were readily discarded. Combinations based on CA:LIM (1:1) (example provided for comparative purposes and outside the scope of the claims), menthol:LIM (1:1) (example provided for comparative purposes and outside the scope of the claims), IBU:LIM (1:4) and IBU:LIM (1:8) were the ones that succeed in the formation of a transparent liquid solution which indicates the loss of lattice arrangement.

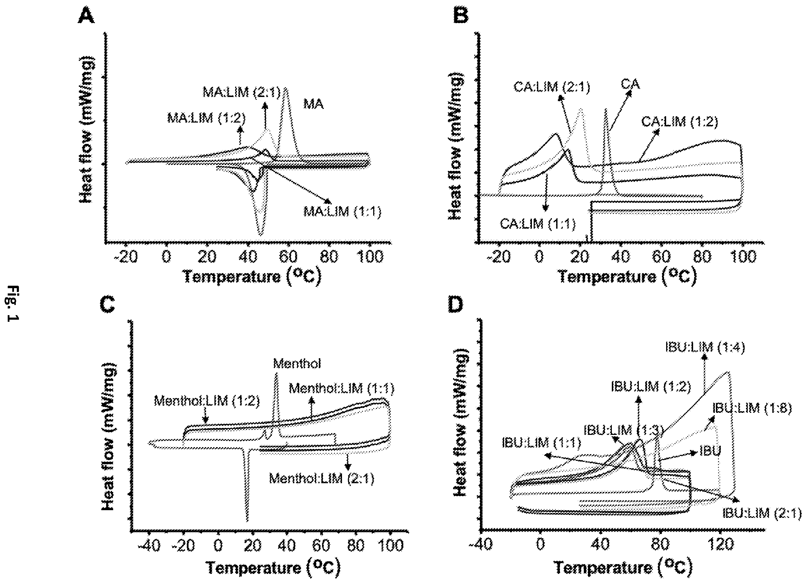

In another embodiment, thermal behaviour was evaluated. The evaluation of the thermal behaviour confirmed the formation of a eutectic mixture (Figure 1), since the thermograms of the eutectic mixtures does present the sharp endothermic peaks of the pure starting components, showing a depression on the melting peak of the individual components when in THEDES form. The presence of peaks of the counterparts indicate that the molecules are not involved in the intermolecular interactions being those formulations readily discarded.

The hypothesis of having similar or different effects between THEDES and a simple mixture of the compounds has also been raised.

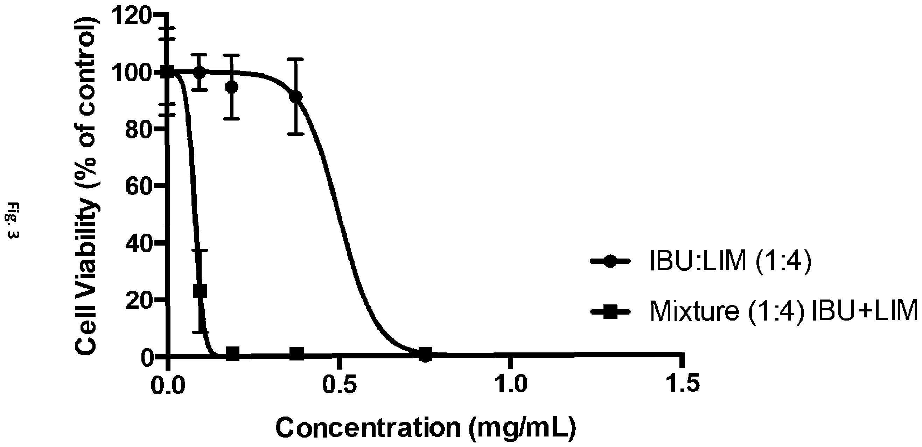

In one embodiment, a direct mixture of IBU with LIM in culture medium was performed and antiproliferative activity assessed, where it showed to be totally different from IBU:LIM (1:4) (Figure 3), confirming once more that IBU:LIM (1:4) is a eutectic mixture, different from a mixture of IBU and LIM. These screening tests allow the preparation of systems where it will be possible to take advantage of the interactions effect between the individual components. Furthermore, when in the THEDES form, IBU presents an increase in its solubility of 4.32-fold as compared to IBU in powder form. An increase in the solubility of IBU prevents its precipitation and coagulation, which is a step forward to accomplish desired pharmacological responses.

In another embodiment, the anticancer properties of IBU:LIM (1:4) were evaluated analysing the effects on cell cycle, apoptosis, intracellular reactive oxidative species ("ROS") and nitric oxide ("NO") production.

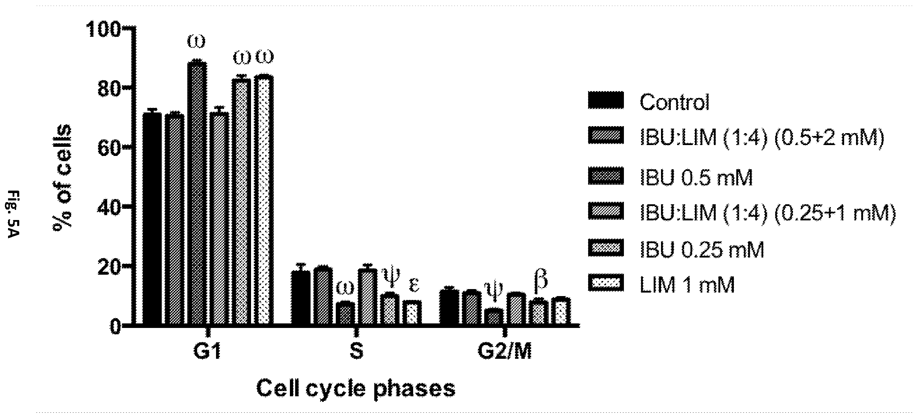

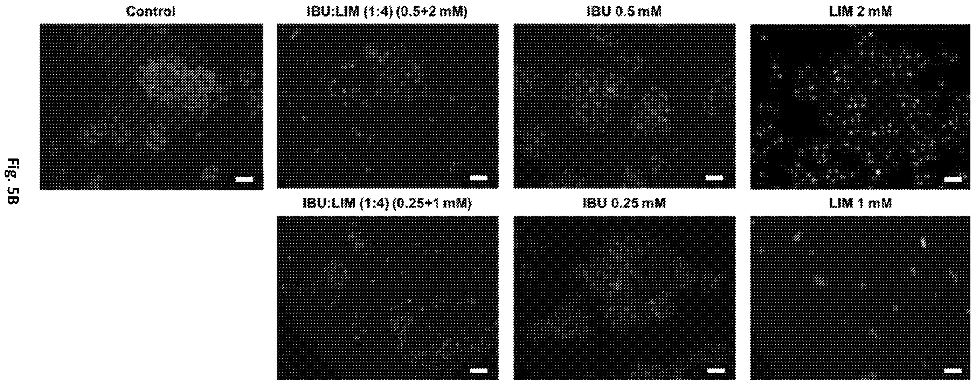

In one embodiment, IBU:LIM (1:4) was not capable of inhibiting cell cycle or apoptosis. This suggests that the antiproliferative effect of IBU:LIM (1:4) may not be due to the cell death mechanism involving caspase-3 dependent apoptosis or cell cycle arrest. Assessing intracellular ROS and NO production, the results indicate that the lowest concentration of IBU:LIM (1:4) (0.25 + 1 mM) protected HT29 cells from oxidative stress, demonstrating anti-inflammatory effects by inhibiting ROS and NO production (Figure 5A and 5B). Inflammation is a process intrinsically associated with carcinogenesis and, consequently, NO levels were determined since they play an important role in modulating the inflammatory molecular pathways which is highly increased in human colonic mucosa. Moreover, NO production has also been related with programmed cell death by inappropriate production of NO which leads to oxidative stress. Herein, the results showed a concentration dependence of IBU:LIM (1:4), where the highest concentration (0.5 + 2 mM) of THEDES promoted NO production above the basal intracellular level (although decreasing intracellular ROS level), leading to cell death.

In one embodiment, the apoptosis was not induced by caspase-3 cascade as shown in Figure 5B. This suggests that this process should be induced via caspase-2 or caspase-9 pathways. As a NSAIDs, IBU has anti-inflammatory and antiproliferative properties which can lead to cell cycle arrest and induction of apoptosis. The present results corroborated with the ones in the literature; any differences in results might be due to the different concentrations herein tested as the different effects according to each cell line.

Ibuprofen has been reported as a compound easily absorbed by passive transport associated to pH gradient at the intestinal gut. Terpenes, such as LIM, are known to stimulate paracellular transport of bigger molecules by interacting with tight junctions, however, its own transport is mainly passive.

In one embodiment, the eutectic composition of IBU:LIM surprisingly inhibit cancer proliferation without compromising the viability of other cells.

In one embodiment, transportation studies were performed using Caco-2 cell model differentiated in a transwell plate. The maintenance of Transepithelial Electrical Resistance (TEER) values reinforces the non-cytotoxicity of the concentration applied. The results obtained indicate a transcellular transport of IBU:LIM (1:4). While 40 % of the DES was observed in either the basal or apical side, the remaining 60% was accumulated inside the cells or metabolized, which provides an indication that cells are able to metabolize or retain the DES in their interior. Moreover, this can confirm the effectiveness IBU:LIM (1:4) action.

In one embodiment, the results suggest that IBU:LIM (1:4) has different effects depending on the dose, being the mechanism of action completely different from isolated IBU and LIM. Therefore, these evidences demonstrate the effectiveness of the IBU:LIM (1:4) as compared to the individual compounds. Indeed, this THEDES comprises the protective and anti-inflammatory properties of IBU and the anticancer properties of LIM. Thus, it was surprisingly observed that using IBU:LIM (1:4) it is possible to decrease the cell cytotoxicity associated with LIM and increase the solubility of IBU. This shows the potential of this system as a drug delivery system.

### Brief Description of the Drawings

The following figures provide preferred embodiments for illustrating the description and should not be seen as limiting the scope of invention.
**Figure 1** - Differential Scanning Calorimetry (DSC) thermograms of THEDES based on limonene. Peaks arising above the baseline represent endothermic peaks.

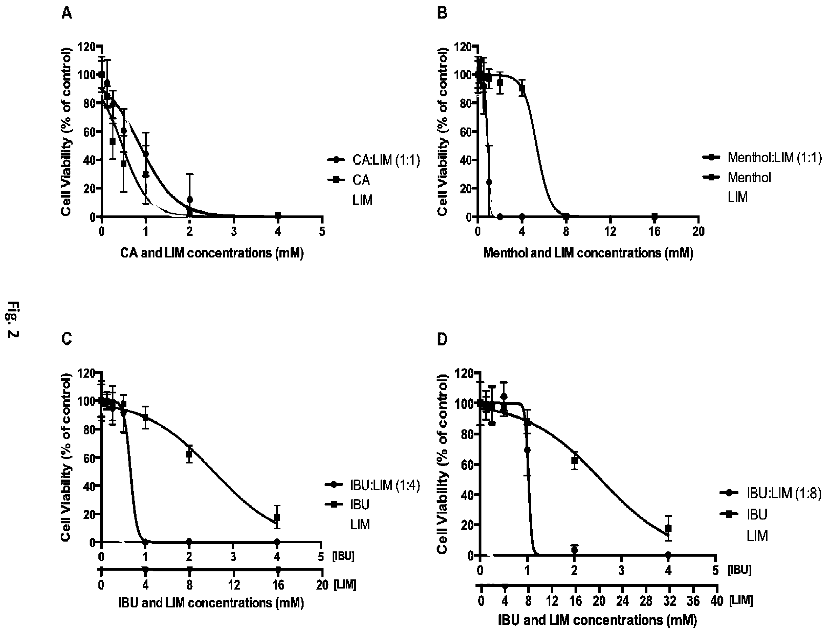

**Figure 2** - Antiproliferative effect of CA:LIM* (1:1) (A), menthol:LIM* (1:1) (B), IBU:LIM (1:4) (C) and IBU:LIM (1:8) (D) using HT29 cell model treated for 24 hours. Results were expressed relative to the control as mean ± SD of at least three independent experiments performed in triplicate (Example provided for comparative purposes and outside the scope of the claims.
**Figure 3** - Comparing antiproliferative effect of THEDES and mixture of IBU and LIM. IBU:LIM (1:4), and a mixture of IBU and LIM were compared in terms of antiproliferative activity using HT29 cell model treated for 24 hours. Results were expressed relative to the control as mean ± SD of three independent experiments performed in triplicate.

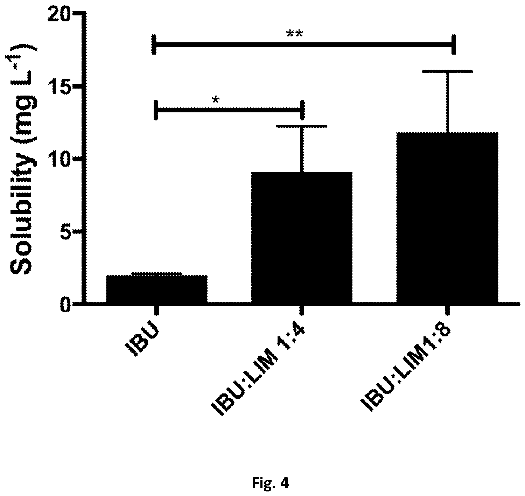

**Figure 4** - Solubility of IBU in powder form or complexed in THEDES in Hank's Balanced Salt Solution (HBSS solution) at physiologically like conditions (pH 7.4., 37°C).
**Figure 5** - Effect of IBU:LIM (1:4), IBU and LIM in inducing cell cycle arrest and apoptosis on HT29 cells. Figure 5A shows the cell cycle analysis distribution in HT29 cells after incubation for 24 hours. Figure 5B shows Caspase-3 detection in HT29 cells treated during the 24 hours. Active caspase-3 was detected by incubation with NucView488TM (apoptotic cells in green) and viable cells were detected by incubation with MitoView633TM (cells with active mitochondria in red). The scale bar is 50 µm. Results are mean ± SD of four (Figure 5A) and two (Figure 5B) independent experiments performed in duplicated. Statistically significant differences are expressed as asterisks (β=P≤0.05, ε=P≤0.01, ψ=P≤0.001, ω=P≤0.0001) in one-way ANOVA analysis for multiple comparisons by Dunnett's method.

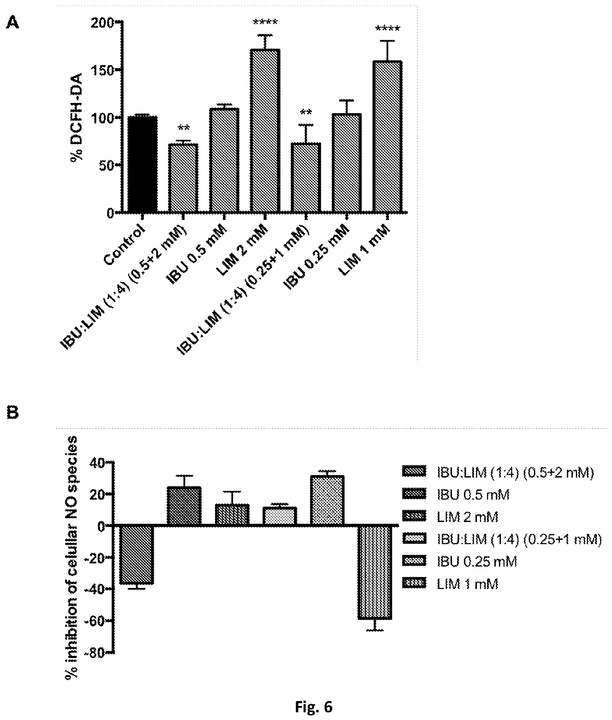

**Figure 6** - Effect of IBU:LIM (1:4), ibuprofen and limonene on ROS accumulation (Figure 6A) and NO production (Figure 6B) using HT29 cell model with 24 hours treatment. Results were expressed relative to the control as mean ± SD of three independent experiments performed in triplicate. Statistically significant differences were analysed according to one-way ANOVA for multiple comparisons by Dunnett's method (**=P≤0.01, ****=P≤0.0001).

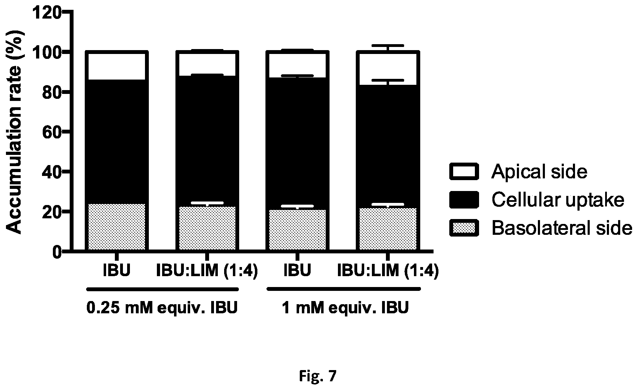

**Figure 7** - IBU:LIM (1:4) cell transport in differentiated Caco-2 transwell cell model. Results were expressed in terms of percentage of accumulation rate at the apical and basolateral sides of the transwell plate and cellular uptake of three independent experiments performed in duplicate. Statistically significant differences as compared to the same concentration of pure IBU were calculated according to one-way ANOVA for multiple comparisons by Dunnett's method.

### Detailed Description

The present disclosure is further described, in particular, using embodiments of the disclosure. Therefore, the disclosure is not limited to the descriptions and illustrations provided. These are used so that the disclosure is sufficiently detailed and comprehensive. Moreover, the intention of the drawings is for illustrative purposes and not for the purpose of limitation.

In an embodiment, different THEDES based on limonene (LIM) were developed to unravel the anticancer potential of such systems.

In one embodiment, THEDES mixtures were prepared. The reagents used in the preparation of THEDES were S-Limonene (LIM, ref.218367 Sigma Aldrich), Ibuprofen (IBU, ref. I4883, Sigma Aldrich), Capric acid (CA, ref. A14788.30, Sigma Aldrich), and menthol (ref.M2772, Sigma Aldrich). The LIM:IBU, CA:LIM (example provided for comparative purposes and outside the scope of the claims), menthol:LIM systems (Example provided for comparative purposes and outside the scope of the claims) were prepared at different molar ratios. The systems were prepared by gently mixing the two components at the given molar ratio. The mixture was heated to 40°C, with constant stirring, until a clear liquid solution was formed.

In one embodiment, thermal properties were evaluated using Differential Scanning Calorimetry (DSC). The DSC experiments were performed in a thermal analyser ("TA") DSC Q100 model (Thermal analysis & analysers, USA), using the different formulation in a TA aluminium pan. The temperature program for CA and CA:LIM (example provided for comparative purposes and not within the scope of the claimed invention) comprises a heating step from -20°C to 100°C at 5°C min⁻¹, an isothermal step of 2 min at 100°C and a cooling step to 20°C at 5°C min⁻¹. For menthol thermograms the samples were equilibrated at 40°C for 5 min followed by cooling to -40°C, an isothermal period for 5 min and heating to 120°C at 5°C min⁻¹. The temperature programme for IBU based THEDES comprise a heating step from -20°C to 120°C at 5°C min⁻¹, an isothermal step of 2 min at 120°C and a cooling step from 120°C to 20°C at 5°C min⁻¹. All measurements were performed under a nitrogen atmosphere (purge gas flux of ca. 50 mL min⁻¹).

In one embodiment, solubility measurements were performed using IBU in powder or THEDES form (IBU:LIM at a molar ratio of 1:4 and 1:8). Briefly, an excess of API in powder and in THEDES form was added to a Hank's Balanced Salt Solution (HBSS, ref 14025-092, Alfagene) solution (≈37°C) and stirred for 24h. The determination of the API solubility was quantified by UV-vis spectroscopy at 265 nm in a microplate reader (BIO-TEK, SYNERGY HT).

In one embodiment, human Caco-2 and HT29 cell lines were obtained from Deutsche Sammlung von Mikroorganismen und Zellkulturen (DSMZ, Germany) and American Type Culture Collection (ATCC, USA), respectively. These cell lines were cultured in RPMI 1640 medium supplemented with 10% of heat-inactivated Fetal Bovine Serum (FBS). In the case of Caco-2, 1% Penicillin-Streptomycin (PS) was also added to the medium. Cells were maintained at 37°C with 5% CO₂ in a humidified incubator and routinely grown as a monolayer in 75 cm² culture flasks. The cell culture medium and supplements were purchased from Invitrogen (Gibco, Invitrogen Corporation, UK).

In one embodiment, cytotoxicity assay was performed using confluent and non-differentiated Caco-2 cells. The assay was performed as previously described by Rodrigues et al. Briefly, Caco-2 cells were seeded into 96-well plates at a density of 2x10⁴ cells/well and allowed to grow for 7 days, with medium renewal every 48 hours. On day 7, cells were incubated with the samples diluted in culture medium. Cells incubated only with culture medium were considered as control. After 24 hours, cells were washed once with PBS (Sigma-Aldrich, USA) and cell viability was assessed using CellTiter 96^{®} AQueous One Solution Cell Proliferation Assay (Promega, USA) containing MTS reagent, according to manufacturer's instructions. The absorbance was measured at 490 nm using a Spark^{®} 10M Multimode Microplate Reader (Tecan Trading AG, Switzerland) and cell viability was expressed in terms of percentage of living cells relative to the control. At least three independent experiments were performed in triplicate.

In one embodiment, the antiproliferative effect of THEDES and standard compounds was evaluated in HT29 cells. Briefly, cells were seeded at a density of 1x10⁴ cells/well in 96-well culture plates. After 24 hours cells were incubated with different concentrations of the samples diluted in culture medium. Cells incubated only with culture medium were considered as control. Cell proliferation was measured after 24 hours using MTS reagent, as mentioned above. Results were expressed in terms of percentage of living cells relative to the control. At least three independent experiments were performed in triplicate.

In one embodiment, cell transport studies were conducted. Caco-2 cells were seeded in 12 mm i.d. Transwell^{®} inserts (polycarbonate membrane, 0.4 µm pore size, Corning Costar Corporation) in 12-well plates at a density of 2.24x10⁵ cell/mL. Cells were allowed to grow and differentiate to confluent monolayers for 21-24 days post seeding by changing the medium three times a week. Transepithelial Electrical Resistance (TEER) of grown cells in Transwell was measured using EVOM^{™} voltmeter (WPI, Germany). Only monolayers with a TEER value higher than 700 Ωcm² were used for experiments. For the assays, cells were washed two times with HBSS and the compounds diluted in HBSS were added to the apical side. Transepithelial transport was followed at several time points (0, 15, 30, 60, 120, 180, 240 minutes and 24 hours) where 200 µL was collected from the basolateral side. Samples were analysed by HPLC method as described above and concentration of compounds calculated. Results were expressed in terms of percentage of compounds at basolateral and apical sides and uptake by the cells. Three independent experiments were performed in duplicate.

In one embodiment, High Performance Liquid Chromatography ("HPLC") analysis of samples resulting from transepithelial transport studies were performed using a Waters Alliance HPLC system (2695, Waters, Milford, MA, USA) coupled to a photodiode array detector (2996, Waters, Ireland). Separation was carried out in a reversed-phase column LiChrospher 100 RP-18 (5 µm) LiChroCART 250-4 (Merck Millipore, Kenilworth, NJ, USA) in isocratic mode, with a mobile phase formed by 60:40 (%v/v) acetonitrile:water acidified with phosphoric acid (pH 2.5). The injection volume was set at 40 µL, the flow rate at 1.0 mL/min and the column temperature at a constant temperature of 40°C. Transepithelial transport was followed as a function of time by detection of IBU at 221 nm. Empower Pro (2002) software was used for data acquisition.

In one embodiment, cell cycle assessment was conducted. HT29 cell line was seeded at a density of 1x10⁶ cells in a 25 cm² culture flask for 24 hours. Then, cells were incubated another 24 hours with different concentrations of the samples diluted in culture medium and culture medium alone (as control). For flow cytometry analysis of DNA content, bare nuclei were prepared. Briefly, cells were detached from the culture flasks with trypsin-EDTA 0.5% and washed one time with cold PBS. Finally, 1x10⁶ cells were re-suspended in 1 mL of staining solution containing 50 mg/mL of Propidium Iodide (Sigma-Aldrich, USA), 1.5% (v/v) of Triton X-100 (Sigma-Aldrich, USA), 0.700 U/mL of DNase/protease-free Ribonuclease A (Thermo Scientific, USA) and 0.01 M of NaCl, followed by incubation at room temperature in the dark for 2 hours. Cell cycle was assessed by flow cytometry, using a CyFlow space (Partec GmbH) instrument, registering 30.000 events/sample.

In one embodiment, apoptosis was evaluated by caspase-3 activity using NucView488TM and MitoView633TM Apoptosis Assay Kit (Biotium, USA) that allows staining of living cells by a far-red fluorescent dye MitoView633TM and the intracellular caspase-3/7 activity and cells nuclei morphological changes by bright green fluorescent dye NucView488TM. HT29 cells were seeded at a density of 40.000 cell/cm² at 24-well plate for 24 hours. Then, cells were incubated another 24 hours with different concentrations of the samples diluted in culture medium and in culture medium alone (as control). Staining was performed with 200 µL/well of culture medium containing 1 µL of NucView488TM and 1 µL of MitoView633TM for 2 hours. Cells were observed under fluorescence microscope (Leica DM6000, Germany) and image analysis performed using ImageJ software.

In one embodiment, intracellular ROS level were assessed according to the method described by Wolf and Liu (2007) with slight alterations. HT29 cells were seeded at a density of 40.000 cell/cm² in a 24-well plate. After 24 hours, cells were incubated for 1 hour with different concentrations of the samples diluted in culture medium and in culture medium alone (as control). Cells were then washed two times with PBS, thereafter 600 uL of dichlorofluorescin-diacetate (DCFH-DA 25 uM) was added and the cells were allowed to incubate for 1 hour. Fluorescence was measured using a Microplate Fluorimeter FLx800 (Bioteck Instruments) (excitation and emission wavelengths of 485 nm and 528 nm, respectively). Results were expressed in terms of percentage of fluorescence intensity relative to the control. Three independent experiments were performed in triplicate.

In one embodiment, Griess assay was conducted. In order to evaluate the inhibition of nitric oxide (NO) species produced by HT29 treated with THEDES or isolated compounds, culture media was assessed using Griess reaction. A mixture of 50:50 (v/v) of Griess reagent (1% sulphanilamide, 0.1% N-(L-naphthyl)-ethylene diamine dihydrochloride and 2% H₃PO₄) and cell supernatant was prepared and the absorbance of the coloured solution was measured at 540 nm using a Spark^{®} 10M Multimode Microplate Reader (Tecan Trading AG, Switzerland). Percentage of NO produced was expressed relative to the control. Three independent experiments were performed in triplicate.

In one embodiment, all data were expressed as mean ± Standard Deviation (SD). GraphPad Prism 6 software was used to calculate EC₅₀ values (the concentration of sample necessary to decrease 50% of cell population) and to analyse significant differences between data set through One-Way Analysis of Variance (ANOVA) following Dunnett's multiple comparison tests. A p-value < 0.05 was considered statistically significant.

In one embodiment, THEDES were prepared by gently mixing capric acid (CA), menthol or ibuprofen (IBU) with LIM. Successful eutectic mixtures were obtained for menthol:LIM (1:1) (example provided for comparative purposes and outside the scope of the claims), CA:LIM (1:1) (example provided for comparative purposes and outside the scope of the claims), IBU:LIM (1:4) and IBU:LIM(1:8). LIM-based THEDES were evaluated in terms of their cytotoxicity and antiproliferative effects. The results indicate that all the THEDES present antiproliferative properties, but IBU:LIM; preferably IBU:LIM (1:4), surprisingly inhibit HT29 proliferation without compromising cell viability. Therefore, IBU:LIM (1:4) was the formulation selected for further assessment of anticancer properties. The results suggest that the mechanism of action of LIM:IBU is different from isolated IBU and LIM. Thus, the eutectic composition, IBU:LIM comprises the protective and anti-inflammatory properties of IBU allied to the anticancer properties of LIM.

In one embodiment, a novel THEDES based on LIM was developed due to LIM's well-known anticancer properties. LIM was combined with different compounds, including menthol, IBU and CA, due to their well-known anti-inflammatory properties. CA also increases the bioavailability of the API. Different formulations based on LIM were prepared. Formulations that led to a pasty-like solid at RT were readily discarded. Combinations based on CA:LIM (1:1) (Example provided for comparative purposes and outside the scope of the claims), menthol:LIM (1:1) (Example provided for comparative purposes and outside the scope of the claims), IBU:LIM (1:4) and IBU:LIM (1:8) were the ones that succeed in the formation of a transparent liquid solution. This indicates the loss of lattice arrangement. The evaluation of the thermal behaviour confirmed the formation of a eutectic mixture (Figure 1). The initial thermograms showed sharp endothermic peaks of the pure starting compounds, thereafter the thermograms showed a depression in the melting peak of the individual compounds when THEDES form.

In one embodiment, LIM was mixed with different compounds (i.e. myristic acid (MA), menthol, CA, IBU) to obtain novel eutectic formulations with pharmaceutical applications that may boost the use of THEDES in cancer treatments. The different molar ratios are listed in Table 1. Using CA:LIM (example provided for comparative purposes and outside the scope of the claims) at a molar ratio of 1:1; 1:2 and 2:1 a clear and transparent liquid without any precipitate was obtained at RT after 1-2 hours. Similarly, a clear liquid was obtained using menthol:LIM (example provided for comparative purposes and outside the scope of the claims) at a molar ratio of 1:1, 1:2 and 2:1 where it was observed that there were no insoluble particles visible to the naked eye. Combining IBU with LIM in different molar ratios, only 1:8 gives rise to a transparent liquid at RT, whereas using a molar ratio of 1:4, a liquid mixture with almost imperceptible crystals was obtained at RT.

**Table 1: Summary of the different THEDES prepared.**

| **THEDES** | **Molar Ratio** | **Visual aspect at RT** | **Melting Point (°C)** |
|---|---|---|---|
| **MA:LIM*** | 1:1 | Solid | ≈47.7 |
| | 1:2 | Solid | ≈47.5 |
| | 2:1 | Solid | ≈51.6 |
| **CA:LIM*** | 1:1 | Liquid | ≈14.3 |
| | 1:2 | Liquid | ≈7.8 |
| | 2:1 | Liquid | ≈20.9 |
| **Menthol:LIM*** | 1:1 | Liquid | - |
| | 1:2 | Liquid | - |
| | 2:1 | Liquid | - |
| **IBU:LIM** | 1:1 | Solid | ≈58.4 |
| | 2:1 | Solid | ≈59.8 |
| | 1:2 | Solid | ≈63.05 |
| | 1:3 | | |
| | 1:4 | Liquid with few crystals | ≈29.8 |
| | 1:8 | | - |
| | | Liquid | |

| | | | |
|---|---|---|---|
| * Example provided for comparative purposes and outside the scope of the claims.In one embodiment, the eutectic formulations were further studied by Differential Scanning Colorimetry ("DSC"). The DSC analysis (Figure 1) of CA:LIM (example provided for comparative purposes and outside the scope of the claims) spectra present a unique and well-defined peak at ≈ 7.8-20.9°C indicating a depression in the original melting point of CA at ≈ 32°C (Figure 1A). On the other hand, the spectra of menthol:LIM (example provided for comparative purposes and outside the scope of the claims) does not present any peak (Figure 1B). Likewise, LIM was also mixed with IBU at different molar ratios and the thermograms were dominated by the presence of sharp endothermic peaks, that are ascribed to IBU (i.e., molar ratios from 1:1 to 1:3). Contrarily, using a molar ratio of 1:4 and 1:8 a strong depression on the melting point of IBU was observed. | | | |

In one embodiment, the cytotoxicity and antiproliferative effects of LIM-based THEDES were assessed (Figure 2). LIM-based THEDES were evaluated in terms of their cytotoxicity and antiproliferative effect. As Figure 2 shows, CA:LIM (1:1) (Example provided for comparative purposes and outside the scope of the claims), menthol:LIM (1:1) (Example provided for comparative purposes and outside the scope of the claims), IBU:LIM (1:4) and IBU:LIM (1:8) systems inhibited HT29 proliferation in a dose-dependent manner, being CA:LIM (1:1) (example provided for comparative purposes and not within the scope of the claimed invention) system the one with lowest EC50 value (0.6901 ± 0.105 mM of equivalent limonene, Table 2).

**Table 2: EC₅₀ values obtained from cytotoxicity and antiproliferative assays of isolated compounds and THEDES in Caco-2 and HT29 cells, respectively. Results were expressed in mean ± SD of equivalents of LIM.**

| | **EC₅₀ values (mM)** | |
|---|---|---|
| **Isolated compounds** | **Cytotoxicity assay** | **Antiproliferative assay** |
| IBU | 2.893 ± 0.059 | 2.346 ± 0.088 |
| CA | 1.334 ± 0.223 | 0.341 ± 0.081 |
| LIM | 2.638 ± 0.108 | 0.661 ± 0.025 |
| Menthol | 8.078 ± 0.810 | 4.730 ± 16.14 |

| **THEDES** | *equivalents of Limonene (mM)* | |
|---|---|---|
| CA:LIM (1:1)* | 0.918 ± 0.042 | 0.6901 ± 0.105 |
| Menthol:LIM (1:1)* | 2.314 ± 0.421 | 0.8023 ± 0.016 |
| IBU:LIM (1:4) | 10.50 ± 0.883 | 2.390 ± 2.919 |
| IBU:LIM (1:8) | 3.323 ± 0.228 | 1.137 ± 0.055 |

| | | |
|---|---|---|
| *Example provided for comparative purposes and outside the scope of the claims. | | |

In one embodiment, CA:LIM (1:1) (example provided for comparative purposes and outside the scope of the claims) and menthol:LIM (1:1) systems (example provided for comparative purposes and outside the scope of the claims) showed similar antiproliferative activity as compared to isolated LIM (Table 2). Using IBU:LIM (1:4) and IBU:LIM (1:8) eutectic systems, a behaviour distinct from the single components was observed. Although all eutectic mixtures showed antiproliferative effect, only IBU:LIM (1:4) was selected for further bioactivity evaluation. IBU:LIM (1:4) antiproliferative effect was compared with the simple mixture of IBU and LIM without THEDES preparation.

As Figure 3 shows, the mixture has higher antiproliferative effect (EC₅₀=0.074 ± 0.006 mg/mL) and a clearly different behaviour from IBU:LIM (1:4) THEDES (EC₅₀=0.4489 ± 0.548 mg/mL).

In one embodiment, the potential of LIM based THEDES to increase the solubility of IBU was assessed. As a non-steroidal anti-inflammatory compound, IBU has a very low solubility in water (≈ 2mg mL⁻¹). Thus, the solubility of IBU in physiological-like condition (i.e., HBSS at 37°C) in powder and THEDES form was quantitatively determined (Figure 4). The results clearly indicate an increase in IBU solubility by 4.32-fold (IBU:LIM (1:4)) and 5.63-fold (IBU:LIM (1:8)), when compared with IBU in powder form.

In one embodiment, the bioactivity of THEDES was evaluated. Two non-cytotoxic concentrations of IBU:LIM (1:4) were used for analysis of cell cycle, apoptosis, intracellular ROS and NO production. As Figure 2 shows, IBU:LIM (1:4) was not capable of inhibiting cell cycle or apoptosis, whereas isolated IBU and LIM promoted cell cycle arrest in G1-phase (Figure 5A). Additionally, LIM at 2 mM was capable of inducing apoptosis via caspase-3 activity (Figure 5B).

In one embodiment, the effect over oxide species production was assessed. IBU:LIM (1:4) was capable of protecting HT29 cells from oxidative stress by decreasing the production of basal intracellular ROS relatively to the control (Figure 6A). Figure 6B shows that the highest concentration of IBU:LIM (0.25 + 2mM) induced the production of NO, whereas the lowest concentration of THEDES (0.25 + 1 mM) protected HT29 cells from oxidative stress.

In one embodiment, the selected combinations of THEDES were used to assess cell cytotoxicity and antiproliferative effects. The similar behaviour between LIM and CA:LIM (1:1) (example provided for comparative purposes and outside the scope of the claims) and menthol:LIM (1:1) (example provided for comparative purposes and outside the scope of the claims) (Figure 2A and 2B, Table 2) suggests that the effect of these THEDES is mainly due to the action of LIM itself. LIM has been reported in the literature as a compound with antiproliferative properties where it is able to inhibit colorectal cancer cell growth. On the other hand, IBU:LIM (1:4) and IBU:LIM (1:8) systems (Figure 2C and 2D) have IBU which might have led to the decrease of the antiproliferative activity of the systems comparing to LIM. Although, the results indicate that all the THEDES presented antiproliferative properties, IBU:LIM (1:4) was the only system able to inhibit HT29 proliferation without compromising cell viability (i.e., with the lowest cytotoxicity for normal colonic cells (Caco-2 model)), as shown in table 2. Therefore, IBU:LIM (1:4) was the formulation selected for further assessment of anticancer properties.

In one embodiment, the effect of IBU:LIM (1:4), ibuprofen and limonene on ROS accumulation (Figure 6A) and NO production (Figure 6B) using HT29 cell model with 24 hours treatment was analysed. Results were expressed relative to the control as mean ± SD of three independent experiments performed in triplicate. Statistically significant differences were analysed according to one-way ANOVA for multiple comparisons by Dunnett's method (**=P≤0.01, ****=P≤0.0001).

In one embodiment, IBU:LIM (1:4) cell transport in differentiated Caco-2 transwell cell model was analysed (Figure 7). Results were expressed in terms of percentage of accumulation rate at the apical and basolateral sides of the transwell plate and cellular uptake of three independent experiments performed in duplicate. Statistically significant differences as compared to the same concentration of pure IBU were calculated according to one-way ANOVA for multiple comparisons by Dunnett's method.

In an embodiment, other TEDES prepared combining an NSAIDs and a terpene can be obtained, as listed on Table 3. The systems were prepared by gently mixing the two components at the given molar ratio. The mixture was heated to 40°C, with constant stirring, until a clear liquid solution was formed. From the table it can be observed that different combinations were successfully obtained using ibuprofen, flurbiprofen and ketoprofen. In which concerns acetylsalicylic acid, fewer combinations were successfully achieved. In a further embodiment, the cytotoxicity (Caco-2 cell line) and antiproliferative effects (HT29 cells) of said THEDES, prepared from NSAIDs and terpenes, were assessed (Table 4).

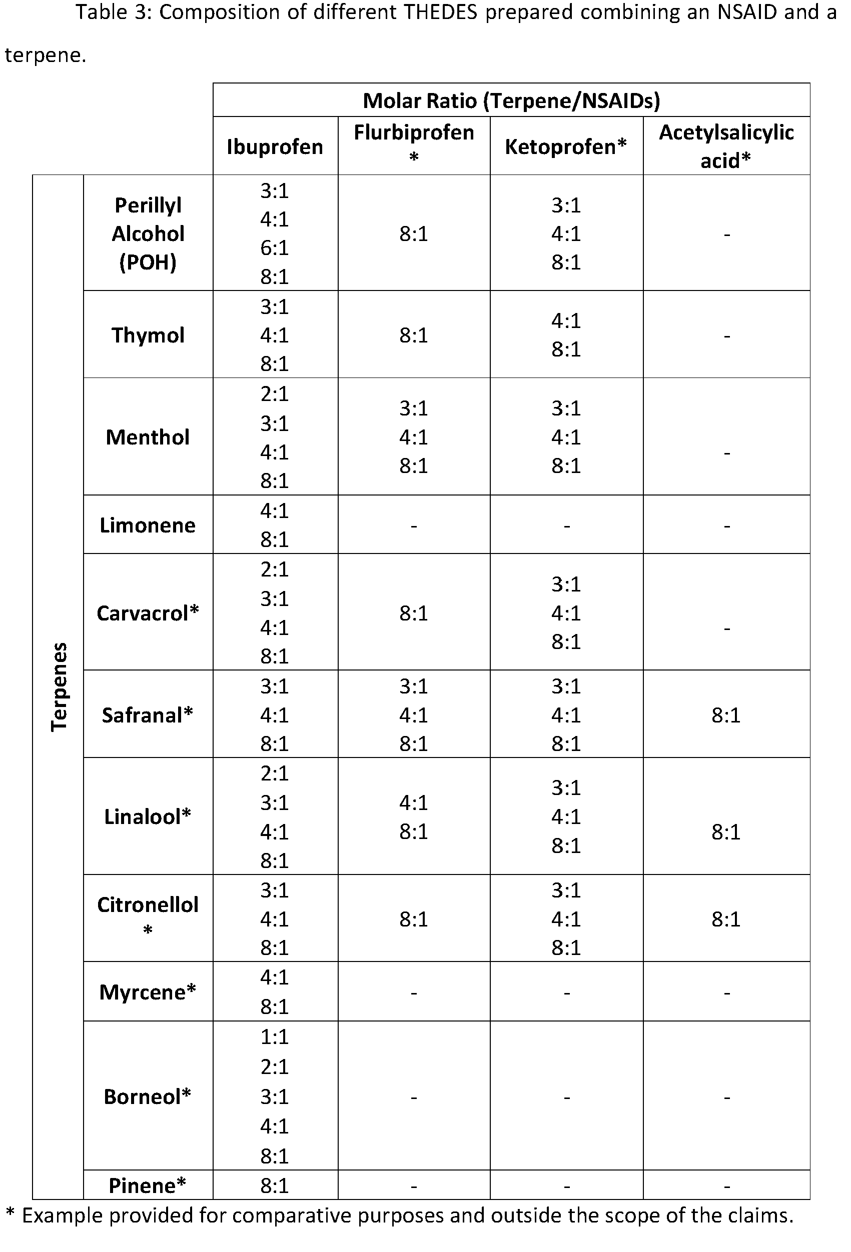

**Table 4: EC50 of the different systems tested, obtained from cytotoxicity and antiproliferative assays of THEDES in Caco-2 and HT29 cells, respectively. Results were expressed in mean ± SD.**

| **THEDES** | | | **EC50 (mM)** | |
|---|---|---|---|---|
| **Terpenes** | **NSAIDs** | **Molar Ratio (Terpene/NSAIDs)** | **Citotoxicity** | **Antiproliferative** |
| Safranal | Ibuprofen | 3:1* | 3.83 | |
| | | 4:1* | 4.81 | |
| Limonene | Ibuprofen | 4:1 | 10.50 | 2.39 |
| | | 8:1 | 3.32 | 1.14 |
| POH | Ibuprofen | 3:1 | 8.46 | 1.43 |
| | | 8:1 | 4.35 | 1.68 |
| Thymol | Ibuprofen | 3:1 | 1.33 | |
| Menthol | Ibuprofen | 3:1 | 10.50 | 4.15 |
| | Flurbiprofen | 4:1* | 2.01 | |
| | Ketoprofen | 4:1* | 2.00 | |
| Linalool | | 4:1* | 2.76 | |
| | Flurbiprofen | 4:1* | 2.64 | |

| | | | | |
|---|---|---|---|---|
| * Example provided for comparative purposes and outside the scope of the claims. | | | | |

The above described embodiments are combinable.

The term "comprising" whenever used in this document is intended to indicate the presence of stated features, integers, steps, components, but not to preclude the presence or addition of one or more other features, integers, steps, components or groups thereof.

## Claims

1. A eutectic composition for use in the treatment or therapy of cancer comprising a nonsteroidal anti-inflammatory drug and a terpene, wherein the composition comprises a combination of ibuprofen and limonene; ibuprofen and menthol, ibuprofen and thymol, or ibuprofen and perillyl alcohol, wherein the molar ratio of anti-inflammatory drug:terpene varies from 1:1 - 1:8.

2. The composition for use according to any previous claims wherein the molar ratio of anti-inflammatory drug:terpene varies from 1:3 - 1:6; preferably 1:4 - 1:6.

3. The composition for use according to any previous claims wherein the molar ratio of ibuprofen:terpene varies from 1:4 - 1:6.

4. The composition for use according to any previous claims wherein the molar ratio of anti-inflammatory drug:limonene varies from 1:4 - 1:6.

5. The composition for use according to the previous claim comprising ibuprofen and limonene, or ibuprofen and menthol, or ibuprofen and perillyl alcohol.

6. The composition for use according to the previous claim wherein the molar ratio of ibuprofen:limonene varies from 1:3 - 1:8.

7. The composition for use according to any previous claim wherein the molar ratio of ibuprofen:limonene varies from 1:4 - 1:6.

8. The composition for use according to any of the previous claims wherein the ibuprofen concentration is from 0.15-0.75 mM, preferably 0.25 - 0.5 mM.

9. The composition for use according to any of the previous claims wherein the limonene concentration is from 0.5-3 mM, preferably 1-2 mM.

10. The composition for use according to any of the previous claims wherein said composition is an injectable composition or an oral composition.

## Patentansprüche

1. Eine eutektische Zusammensetzung zur Verwendung bei der Behandlung oder Therapie von Krebs, umfassend ein nichtsteroidales entzündungshemmendes Arzneimittel und ein Terpen, wobei die Zusammensetzung eine Kombination von Ibuprofen und Limonen, Ibuprofen und Menthol, Ibuprofen und Thymol oder Ibuprofen und Perillaalkohol umfasst, wobei das molare Verhältnis von entzündungshemmendem Arzneimittel:Terpen von 1:1 - 1:8 variiert.

2. Die Zusammensetzung zur Verwendung nach einem der vorangehenden Ansprüche, wobei das molare Verhältnis von entzündungshemmendem Arzneimittel:Terpen von 1:3 - 1:6, bevorzugt von 1:4 - 1:6 variiert.

3. Die Zusammensetzung zur Verwendung nach einem der vorangehenden Ansprüche, wobei das molare Verhältnis von Ibuprofen:Terpen von 1:4 - 1:6 variiert.

4. Die Zusammensetzung zur Verwendung nach einem der vorangehenden Ansprüche, wobei das molare Verhältnis von entzündungshemmendem Arzneimittel:Limonen von 1:4 - 1:6 variiert.

5. Die Zusammensetzung zur Verwendung nach dem vorangehenden Anspruch, umfassend Ibuprofen und Limonen oder Ibuprofen und Menthol oder Ibuprofen und Perillaalkohol.

6. Die Zusammensetzung zur Verwendung nach dem vorangehenden Anspruch, wobei das molare Verhältnis von Ibuprofen:Limonen von 1:3 - 1:8 variiert.

7. Die Zusammensetzung zur Verwendung nach einem der vorangehenden Ansprüche, wobei das molare Verhältnis von Ibuprofen:Limonen von 1:4 - 1:6 variiert.

8. Die Zusammensetzung zur Verwendung nach einem der vorangehenden Ansprüche, wobei die Konzentration von Ibuprofen 0,15 - 0,75 mM, bevorzugt 0,25 - 0,5 mM beträgt.

9. Die Zusammensetzung zur Verwendung nach einem der vorangehenden Ansprüche, wobei die Konzentration von Limonen 0,5 - 3 mM, bevorzugt 1-2 mM beträgt.

10. Die Zusammensetzung zur Verwendung nach einem der vorangehenden Ansprüche, wobei die genannte Zusammensetzung eine injizierbare Zusammensetzung oder eine orale Zusammensetzung ist.

## Revendications

1. Une composition eutectique pour utilisation dans le traitement ou la thérapie du cancer comprenant un médicament anti-inflammatoire non stéroïdien et un terpène, dans laquelle la composition comprend une combinaison d'ibuprofène et de limonène; d'ibuprofène et de menthol, d'ibuprofène et de thymol, ou d'ibuprofène et d'alcool périllylique, dans laquelle le rapport molaire du médicament anti-inflammatoire : terpène varie de 1:1 à 1:8.

2. La composition à utiliser selon l'une des revendications précédentes, dans laquelle le rapport molaire du médicament anti-inflammatoire : terpène varie de 1:3 à 1:6; de préférence de 1:4 à 1:6.

3. La composition à utiliser selon l'une des revendications précédentes, dans laquelle le rapport molaire de l'ibuprofène : terpène varie de 1:4 à 1:6.

4. La composition à utiliser selon l'une des revendications précédentes, dans laquelle le rapport molaire du médicament anti-inflammatoire : limonène varie de 1:4 à 1:6.

5. La composition à utiliser selon la revendication précédente comprenant de l'ibuprofène et du limonène, ou de l'ibuprofène et du menthol, ou de l'ibuprofène et d'alcool périllylique.

6. La composition à utiliser selon la revendication précédente, dans laquelle le rapport molaire de l'ibuprofène : limonène varie de 1:3 à 1:8.

7. La composition à utiliser selon l'une des revendications précédentes dans laquelle le rapport molaire de l'ibuprofène : limonène varie de 1:4 à 1:6.

8. La composition à utiliser selon l'une des revendications précédentes, dans laquelle la concentration en ibuprofène est de 0,15 à 0,75 mM, de préférence de 0,25 à 0,5 mM.

9. La composition à utiliser selon l'une des revendications précédentes, dans laquelle la concentration en limonène est de 0,5 à 3 mM, de préférence de 1 à 2 mM.

10. La composition à utiliser selon l'une des revendications précédentes, dans laquelle ladite composition est une composition injectable ou une composition orale.
